# EUROPÄISCHE PATENTSCHRIFT

(11) **EP 2 740 448 B1**
(45) Veröffentlichungstag und Bekanntmachung des Hinweises auf die Patenterteilung: **18.10.2017**
(21) Anmeldenummer: 13004884.6
(22) Anmeldetag: 10.10.2013
(51) Int. Cl.: A61F 13/20

(54) **Verfahren und Vorrichtung zum Herstellen eines Tampons sowie damit hergestellter Tampon**
Method and apparatus for making a tampon and tampon made by this
Procédé et dispositif de fabrication d'un tampon et tampon ainsi fabriqué

(30) Priorität: 05.12.2012 DE 102012023812
(43) Veröffentlichungstag der Anmeldung: 11.06.2014
(73) Patentinhaber: Rauscher Consumer Products GmbH, 1140 Wien (AT)
(72) Erfinder: Pötsch, Ernst, A-2525 Schönau a.d. Triesting (AT); Posch, Karl-Heinz, A-2801 Katzelsdorf (AT); Bierbauer, Hermann, A-2733 Grünbach (AT); Steinlechner, Erik, A-2500 Baden (AT)
(74) Vertreter: Seranski, Klaus

(56) Entgegenhaltungen:
- EP-A1- 2 431 014
- CA-A1- 2 085 923
- DE-U1-202009 012 237
- US-A- 4 341 214

## Beschreibung

Die Erfindung betrifft ein Verfahren zum Herstellen eines in eine Körperhöhle, insbes. in die weibliche Scheide, einführbaren Tampons mit einem im wesentlichen zylinderförmigen Saugkörper, der eine sich in Richtung auf ein axiales Ende des Saugkörpers verjüngende Kuppe aufweist, die mit einer sich in Richtung auf das entgegengesetzte axiale Ende des Saugkörpers erstreckenden Vertiefung in ihrem Scheitelbereich ausgestattet ist, bei dem eine Materialbahn aus saugfähigem Material zu einem Wickel aufgewickelt und der Wickel unter Bildung der Vertiefung zumindest abschnittweise in radialer und/oder axialer Richtung verpreßt wird, und eine Vorrichtung zur Ausführung eines solchen Verfahrens.

Ein mit gattungsgemäßen Verfahren herstellbarer Tampon und ein Verfahren und eine Vorrichtung zu seiner Herstellung sind bspw. aus der DE 20 2009 012237 U1 bekannt.

Tampons werden schon seit langer Zeit zur Aufnahme von Körperflüssigkeit in der Wundversorgung, maßgeblich aber im Zusammenhang mit der weiblichen Menstruation, eingesetzt. Ein einfacher Tampon bestehend aus einem Pfropf aus einem aufsaugfähigem Material, einer Umhüllung aus Gaze, einem Rückholfaden und einem Trichter aus durchlässigem Material ist bereits in der 1936 vom Reichspatentamt herausgegebenen Patentschrift 624 395 beschrieben. Die Entwicklung entsprechender Tampons ist bis heute nicht abgeschlossen. Beispielhaft wird für die einzelnen Entwicklungsschritte Bezug genommen auf die deutsche Patentschrift 804 835 aus dem Jahr 1949, die deutsche Offenlegungsschrift 27 22 802 A1 aus dem Jahr 1977, die deutsche Patentanmeldung 35 19 515 A1 aus dem Jahr 1985, die internationale Patentanmeldung mit der Veröffentlichungsnummer WO 01/24729 A2 aus dem Jahr 2001, die europäische Patentanmeldung mit der Veröffentlichungsnummer 2 298 259 A aus dem Jahr 2010 und die danach veröffentlichte europäische Patentanmeldung 2 431 014 A1.

Schon in der Patentschrift 624 395 wird darauf hingewiesen, daß eine Umkleidung des Saugkörpers erforderlich ist, um zu verhindern, daß Rückstände des faserigen Saugkörpers in der Körperhöhle verbleiben. In der DE 35 19 515 A1 wird ein Verfahren vorgeschlagen, wie ein zur Umhüllung des Saugkörpers dienendes Hüllmaterial auf einen gewickelten Tampon aufgebracht werden kann. Bei dem in dieser Schrift beschriebenen Verfahren wird ein Vliesstoffabschnitt auf einem Watteband fixiert, das Watteband aufgewickelt, das hintere Vliesstoffende auf dem darunterliegenden Vliesstoffmaterial festgelegt und das so erhaltene Vorprodukt verpreßt, um so einerseits eine zuverlässige, dem Heraustreten von Fasern aus der Watte entgegenwirkende Umhüllung bereitzustellen und andererseits eine Ausdehnung des Saugkörpers bei Flüssigkeitseintritt ohne Behinderung durch den Vliesstoff zu ermöglichen. Bei den so hergestellten Tampons wird ein "Ausfusseln" des Saugkörpers zuverlässig verhindert. Allerdings hat es sich gezeigt, daß das Saugvermögen der so erhaltenen Tampons in vielen Fällen nicht vollständig ausgeschöpft werden kann und Flüssigkeit aus der Körperhöhle austritt, obwohl der Saugkern des Saugkörpers noch nicht mit Flüssigkeit gesättigt bzw. vollständig genutzt ist. Diese Mängel konnten auch nicht durch Bereitstellung von sich parallel zur Wickelachse erstreckenden Rillen in der äußeren Begrenzungsfläche des Saugkörpers vollständig beseitigt werden.

Eine Lösung der angesprochenen Probleme wird in der EP 2 298 259 A angeboten. Der in dieser Schrift beschriebene Tampon weist im Scheitelbereich der das Einführende des Tampons bildenden Kuppe eine Vertiefung auf, durch welche die dort aufgegriffene Körperflüssigkeit direkt in den Saugkern des Saugkörpers gelangt, so daß eine frühzeitige Sättigung des äußeren Bereichs des Saugkörpers im Kuppenbereich zuverlässig verhindert werden kann und auch der innere Bereich des Tampons optimal genutzt wird.

Eine ähnliche Lösung wird in der EP 2 431 014 A1 vorgeschlagen. Bei einer ersten Ausführungsform der in dieser Schrift beschriebenen Tampons wird ein mit einem Hüllvlies ausgestattetes Watteband aufgewickelt und dann axial und radial derart verpreßt, daß an einem Ende des so hergestellten Tampons eine mit einer Vertiefung ausgestattete Kuppe entsteht.

Bei einer zweiten Ausführungsform von in der genannten Schrift beschriebenen Tampons wird ein mit einem Hüllvlies beidseits abgedeckter Wattebandstreifen W-förmig gefaltet und radial sowie axial verpreßt. Dadurch wird ein Tampon erhalten, dessen äußere Begrenzungsfläche vollständig von dem Hüllvlies abgedeckt ist. Die Herstellung eines solchen Tampons durch Faltung eines Wattebandabschnitts erweist sich allerdings als problematisch.

Endlich wird bei einer dritten Ausführungsform der in der genannten Schrift beschriebenen Erfindung ein erster Wattebandabschnitt kreuzförmig von einem zweiten Wattebandabschnitt überlagert. Die freiliegenden Endbereiche der Wattebandabschnitte werden hochgeklappt und das so erhaltene kastenförmige Gebilde wird radial und axial verpreßt. Auch dieses Herstellungsverfahren erweist sich als problematisch.

Die CA 2 085 923 A1 beschreibt ein Verfahren zur Herstellung eines Tampons, bei dem der gewickelte Tampon mit einem Hüllvlies abgedeckt wird und die über die axialen Enden des gewickelten Tampons hinausstehenden Enden des Hüllvlieses in die an beiden axialen Enden des Wickels ausgebildeten Öffnungen des Wickels eingeschlagen werden.

Ein alternatives Herstellungsverfahren für einen Tampon, das nicht das Aufwickeln eines Wattebands umfasst, ist in der US 4,341,214 A offenbart.

Angesichts der vorstehend beschriebenen Probleme im Stand der Technik liegt der Erfindung die Aufgabe zugrunde, ein einfach ausführbares Verfahren bereitzustellen, mit dem Tampons erhalten werden, welche unter besonders guter Ausnutzung der Saugfähigkeit verwendet werden können, ohne daß die Gefahr des Ausfusselns besteht.

Erfindungsgemäß wird diese Aufgabe durch eine Weiterbildung der bekannten Verfahren gelöst, die im wesentlichen dadurch gekennzeichnet ist, daß eine etwa senkrecht zur Wickelachse verlaufende Stirnfläche des Wickels vor der Verpressung vollständig mit einem flüssigkeitsdurchlässigen Hüllmaterial abgedeckt wird und vor dem Wickelvorgang ein Rückholfaden um die Materialbahn geschlungen wird und die von dem Rückholfaden umschlungene Materialbahn einen Knoterapparat durchläuft, wobei der Längsüberstand vor dem Durchlaufen des Knoterapparats bezüglich einer etwa kolinear zum Längsrand verlaufenden Faltlinie umgefaltet wird und nach Durchlaufen des Knoterapparats wieder aufgerichtet wird.

Dabei geht die Erfindung auf die Erkenntnis zurück, daß der Zwischenschritt des Abdeckens der Stirnfläche des Wickels vor der Verpressung zum fertigen Tampon ohne übermäßigen apparativen Aufwand zuverlässig durchgeführt werden kann, ohne daß die vollständige Abdeckung der in der Kuppe vorgesehenen Vertiefung des fertigen Tampons durch das Hüllmaterial dadurch beeinträchtigt wird. Durch die Vertiefung in der Kuppe des erfindungsgemäßen Tampons wird die besonders gute Ausnutzung der Saugfähigkeit des Saugkörpers sichergestellt. Durch die Abdeckung des Tampons auch im Bereich der Vertiefung durch das vor der Verpressung auf eine Stirnfläche des Wickels aufgebrachte Hüllmaterial wird das unerwünschte Ausfusseln während der Verwendung zuverlässig unterdrückt und eine glatte Oberfläche am Einführende des Tampons bereitgestellt. Schließlich können die gewünschten Eigenschaften ohne übermäßigen Mehraufwand des Herstellungsverfahrens unter Einsatz des bekannten Wickelverfahrens erhalten werden und ohne daß eine Faltung des saugfähigen Materials erforderlich ist, wie sie in der EP 2 431 014 A1 beschrieben ist, um einen mit einer Vertiefung in der Kuppe ausgestatteten Tampon zu erhalten, bei dem auch die Vertiefung mit einem Hüllmaterial abgedeckt ist.

Ähnlich wie bei dem in der EP 2 298 259 A beschriebenen Verfahren wird auch bei dem erfindungsgemäßen Verfahren zweckmäßigerweise das Hüllmaterial vor dem Wickelvorgang auf der Materialbahn angebracht, insbes. aufgesiegelt, wobei sich das Hüllmaterial vorzugsweise über die gesamte Breite der Materialbahn, aber nur über einen Teil der Länge davon erstreckt und an einem Längsende einzelner zur Bildung der Tampons auf vorgegebene Längen geschnittener Materialbahnabschnitte darüber hinausragt. Zum Erhalt eines zur Abdeckung der etwa senkrecht zur Wickelachse verlaufenden Stirnfläche des Wickels dienenden Hüllmaterialbereichs wird das Hüllmaterial bei dem erfindungsgemäßen Verfahren allerdings unter Bildung eines Längsüberstands über einen etwa senkrecht zur Wickelachse verlaufenden Längsrand der Materialbahn auf die Materialbahn aufgebracht und zweckmäßigerweise daran befestigt, insbes. versiegelt, wobei die Breite des Längsüberstands vorzugsweise etwa 13 bis 63 %, insbes. etwa 20 bis 50 %, der Breite der Materialbahn beträgt.

Wie bei den bekannten Verfahren zum Herstellen von Tampons, wird die Materialbahn zum Erhalt von Bahnabschnitten vorgegebener Länge zur Herstellung jeweils eines Tampons vorzugsweise vor Aufbringen des Hüllmaterials längs senkrecht zu den Längsrändern verlaufenden Schnittlinien geschnitten und das Hüllmaterial unter Bildung eines derart über die etwa senkrecht zum Längsrand verlaufende Schnittlinie überstehenden Querüberstands auf den Bahnabschnitt aufgebracht, daß es nach dem Wickelvorgang eine Außenschicht des Wickels mit auf dem Hüllmaterial aufliegendem Querüberstand bildet. Der Querüberstand kann dann auf die darunterliegende Hüllmaterialschicht aufgesiegelt werden, um so eine stabile Umhüllung des Saugmaterials zu erhalten.

Erfindungsgemäß wird die Materialbahn vor dem Wickelvorgang mit dem Rückholfaden ausgestattet, der die Materialbahn umschlingt. Die freien Enden des die Materialbahn umschlingenden Rückholfadens werden dann mit Hilfe eines von der mit dem Rückholfaden ausgestatteten Materialbahn durchlaufenen Knoterapparats miteinander verknotet. Der Rückholfaden ist üblicherweise längs einem den Kern des Tampons bildenden vorderen und nicht mit Hüllmaterial abgedeckten Bereich der Materialbahn angeordnet. Zur Vermeidung einer Störung des Knotvorgangs durch den Längsüberstand wird dieser bei erfindungsgemäßen Verfahren vor Durchlaufen des Knoterapparats bezüglich einer etwa kolinear zum Längsrand der Materialbahn verlaufenden Faltlinie umgefaltet, so daß er über oder unter der Materialbahn zu liegen kommt, und nach Durchlaufen des Knoterapparats wieder aufgerichtet, so daß auch der folgende Wickelvorgang durch den Längsüberstand nicht beeinträchtigt wird.

Wie vorstehend bereits angedeutet, wird bei erfindungsgemäßen Verfahren eine Stirnfläche des Wickels während oder nach dessen Bildung mit dem Längsüberstand des Hüllmaterials abgedeckt. Zur Herstellung des Wickels wird die Materialbahn mit dem darauf aufgebrachten Hüllmaterial in einer Wickelhülse auf einen Wickeldorn gewickelt. Die Abdeckung des so hergestellten Wickels erfolgt noch innerhalb der Wickelhülse. Das kann einerseits während der Bildung der äußeren Wickellage geschehen. Bei einer anderen Ausführungsform wird zunächst der Wickel einschließlich der äußeren Wickellage vollständig hergestellt und danach die Abdeckung der Stirnfläche des Wickels mit dem Hüllmaterial bewirkt. Durch die Abdeckung der Stirnfläche erst nach dem Wickelvorgang wird erreicht, daß die Abdeckung durch den Wickelvorgang selbst nicht beeinträchtigt oder verzerrt wird. Zur Abdeckung der Stirnfläche des Wickels mit dem Längsüberstand des Hüllmaterials kann der Längsüberstand unter Verwendung einer Faltanordnung, z. B. einer Faltkulisse, kontinuierlich, über eine gewisse Wegstrecke verlaufend, über die Stirnfläche des Wickels gefaltet werden. Dabei kann der Wickel ggf. innerhalb der Wickelhülse um seine Wickelachse gedreht und durch diese Drehung und/oder eine Bewegung des Wickels in einer senkrecht zur Wickelachse verlaufenden Richtung mittels der kontinuierlich arbeitenden Faltanordnung über die Stirnfläche des Wickels gefaltet werden. Der Längsüberstand gelangt dabei in Anlage an die Faltanordnung und wird durch die Bewegung des Wickels in der gewünschten Art und Weise über die Stirnfläche gefaltet.

Alternativ oder zusätzlich kann der Längsüberstand nach Bildung des Wickels mit einer diskret arbeitenden Faltanordnung, wie bspw. durch Falthämmer, Faltfinger, Faltrad, Faltzylinder od. dgl., in ein oder mehreren Schritten über die Stirnfläche des Wickels gefaltet werden. Für eine solche diskrete Faltung des Hüllmaterials über die Stirnfläche des Wickels ist es denkbar, die Faltanordnung an einer oder mehrerer Stopppositionen der Wickelvorrichtung zur Bildung des Wickels vorzusehen.

Bei beiden genannten Faltanordnungen zum Erhalt einer vollständigen Abdeckung der Stirnfläche des Wickels mit dem Hüllmaterial ist es besonders zweckmäßig, wenn die Randbereiche des Hüllmaterials - die Stirnfläche des Wickels überdeckend - einander überlappen. Zur Stabilisierung der Gesamtanordnung können übereinanderliegende Bereiche des die Stirnfläche abdeckenden Längsüberstands miteinander verbunden, insbes. verschweißt und/oder versiegelt, werden.

Ähnlich wie bei den bekannten Verfahren wird nach Verpressung des Wickels ein Formwerkzeug, vorzugsweise eine Dornanordnung einer Kuppenformeinrichtung, zur Bildung der Vertiefung in der Kuppe in die von dem Hüllmaterial abgedeckte Stirnfläche bewegt. Selbstverständlich kann alternativ auch der verpreßte Wickel in das Formwerkzeug zur Bildung der Vertiefung in der mit Hüllmaterial abgedeckten Stirnfläche der Kuppe bewegt werden. Dabei kann das Hüllmaterial auch zur Sicherstellung eines den Eintritt von Flüssigkeit in den Saugkern begünstigenden Kanals von der Wickelspitze durchstoßen werden.

Eine erfindungsgemäße Vorrichtung zur Durchführung eines erfindungsgemäßen Verfahrens mit einer Wickeleinrichtung zum Herstellen eines Wickels aus einer Materialbahn aus saugfähigem Material und einer Preßeinrichtung zum Verpressen des Wickels sowie einer Kuppenformeinrichtung zur Bildung der die Vertiefung aufweisenden Kuppe weist eine Abdeckeinrichtung zum Abdecken einer Stirnfläche des Wickels mit dem Hüllmaterial vor der Verpressung auf und ist gekennzeichnet durch die Merkmale des Patentanspruchs 9. Dabei kann die Zuführeinrichtung derart intermittierend betrieben werden, daß das Hüllmaterial nur auf einen die äußere Windung des Wickels bildenden Bereich der Materialbahn aufgebracht wird, der an einem Längsende des Materialbahnabschnitts mit dem Querüberstand darüber hinausragt, so daß der Querüberstand nach Herstellung des Wickels auf einem dann darunterliegenden Hüllmaterialbahnbereich aufgesiegelt werden kann.
Sowohl die Falt- als auch die Rückführeinrichtung einer erfindungsgemäßen Vorrichtung können unter Einsatz von Druckluft betrieben werden.

Bei einer erfindungsgemäßen Vorrichtung kann die Falteinrichtung ein Faltblech aufweisen, längs dem der Längsüberstand des auf die Materialbahn aufgebrachten und vorzugsweise damit verbundenen Hüllmaterials umgefaltet wird, wenn die Materialbahn an dem Faltblech vorbeigefördert wird. Die Rückführeinrichtung kann ein Führungsblech aufweisen, mit dem der umgefaltete Längsüberstand wieder aufgerichtet wird, wobei der Knoterapparat vorzugsweise zwischen dem Faltblech und dem Führungsblech angeordnet ist. Alternativ oder zusätzlich können auch pneumatische Blasdüsen oder andere ggf. berührungsfrei wirkende Mittel für die Umfaltung bzw. für die Aufrichtung des Hüllmaterials zum Einsatz kommen.

Ähnlich wie bei herkömmlichen Vorrichtungen zum Herstellen von Tampons kann die Wickeleinrichtung eine Wickelhülse und einen in der Wickelhülse bezüglich der Hülsenachse drehbar gelagerten Wickeldorn aufweisen, wobei sowohl die Wickelhülse als auch der Wickeldorn einen sich in Richtung der Hülsenachse erstreckenden Aufnahmeschlitz aufweisen, in den die Materialbahn einführbar ist. Dabei wird zweckmäßigerweise nur ein Materialbahnbereich ohne Hüllmaterial in den Aufnahmeschlitz des Wickeldorns eingeführt, wobei das Hüllmaterial mit dem Längsüberstand erst im Verlauf des Wickelvorgangs auf den Wickeldorn gewickelt wird und so die äußere Wickellage bildet. Üblicherweise wird der in der Wickelhülse auf dem Wickeldorn gebildete Wickel nach Abschluß des Wickelvorgangs durch eine Ausstoßöffnung an einem axialen Ende der Wickelhülse ausgestoßen. Bei herkömmlichen Vorrichtungen wird die Ausstoßöffnung nach Einführen der Materialbahn mit Hilfe eines Abdeckblechs gesichert, um so einem sogenannten Teleskopiereffekt des zu bildenden Wickels entgegenzuwirken. Bei einer erfindungsgemäßen Vorrichtung kann die Ausstoßöffnung nicht ohne Beeinflussung des Längsüberstands verschlossen werden. Sie ist daher durch ein offenes axiales Ende der Wickelhülse gebildet, durch das der Längsüberstand während des Wickelvorgangs axial herausragen kann. Dieses offene Ende wird im folgenden auch Überstandsende genannt. Wenngleich die Wickelhülse erfindungsgemäßer Vorrichtungen ebenso wie der Wickeldorn im wesentlichen kreiszylindermantelförmig ausgeführt werden können, hat es sich zur Vermeidung des Herausteleskopierens des Wickels während und/oder nach dem Wickelvorgang in einigen Fällen als günstig erwiesen, wenn sich der Innendurchmesser der Wickelhülse in Richtung auf das Überstandsende verjüngt. Zusätzlich oder alternativ kann sich auch das dem Überstandsende zugewandte Ende des Wickeldorns erweitern, d. h. sich der Außendurchmesser des Wickeldorns in Richtung auf das Überstandsende vergrößern. Unter Einwirkung axialer Kräfte auf eine äußere Wickellage des in der Wickelhülse gebildeten Wickels läuft diese äußere Lage auf das sich verjüngende Ende auf, ohne daß es zu einem Herausteleskopieren kommt. Ähnlich läuft die innere Wickellage unter Einwirkung axialer Kräfte auf das sich erweiternde Ende des Wickeldorns auf, ohne daß ein Herausteleskopieren beobachtet wird. Damit ist es ohne großen apparativen Mehraufwand oder Umbauarbeiten möglich, das Material einwandfrei zu wickeln und den Hüllmaterialüberstand dennoch störungsfrei für eine nachträgliche Abdeckung einer Stirnfläche des Wickels beizubehalten.

Wie vorstehend bereits im Zusammenhang mit der Erläuterung erfindungsgemäßer Verfahren angesprochen, kann die Abdeckeinrichtung zur Abdeckung der Stirnfläche des Wickels eine kontinuierlich arbeitende Faltanordnung, bspw. eine Faltkulisse, aufweisen, mit welcher der aus der Überstandsöffnung herausragende Längsüberstand derart über die Stirnfläche des Wickels gefaltet wird, daß die Stirnfläche durch den Hüllmaterial-Längsüberstand vollständig abgedeckt wird. Zusätzlich oder alternativ kann die Abdeckeinrichtung eine zum Falten des Längsüberstands über eine Stirnfläche des Wickels ausgelegte diskrete Faltanordnung, wie z. B. Falthämmer, Faltfinger oder Faltdüsen, an einer oder mehreren (Stopp-)Positionen an der Wickeltrommel aufweisen. Zur Sicherung der Abdeckung der Stirnfläche des Wickels kann der Faltanordnung eine zum Verbinden übereinanderliegender Bereiche des die Stirnfläche des Wickels abdeckenden Längsüberstands miteinander ausgelegte Verbindungseinrichtung, vorzugsweise eine Siegeleinrichtung, zugeordnet bzw. nachgeordnet sein.

Wie vorstehend bereits im Zusammenhang mit erfindungsgemäßen Verfahren erläutert, kann die Kuppenformeinrichtung der Preßeinrichtung nachgeordnet sein und ein zur Bildung der Vertiefung in die mit dem Hüllmaterial abgedeckte Stirnfläche des Wickels einführbares Formwerkzeug, insbes. eine Dornanordnung, aufweisen. Dabei kann die Dornanordnung auch zum Durchbohren des Hüllmaterials ausgelegt sein.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine schematische Darstellung eines mit erfindungsgemäßen Verfahren herstellbaren Tampons,
- **Fig. 2**: eine schematische Darstellung einer erfindungsgemäßen Vorrichtung zum Herstellen erfindungsgemäßer Tampons und
- **Fig. 3**: schematische Darstellungen von zum Herstellen erfindungsgemäßer Tampons ausgelegten Preßspitzen.

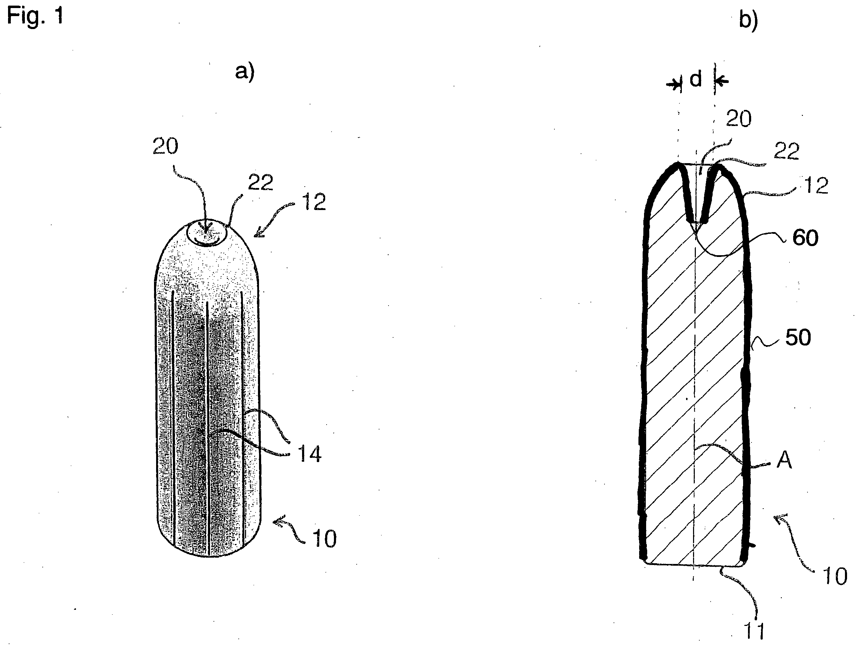

Gemäß Fig. 1 umfaßt der mit erfindungsgemäßen Verfahren herstellbare Tampon einen insgesamt mit 10 bezeichneten, im wesentlichen zylindrischen gewickelten Saugkörper, der an einem seiner axialen Enden eine sich in Richtung auf ein axiales Ende 11 davon verjüngende Kuppe 12 aufweist.

Der Saugkörper 10 weist, wie besonders deutlich der Fig. 1a) zu entnehmen ist, in seiner Mantelfläche eine Anzahl von parallel zueinander und parallel zur Zylinderachse des Saugkörpers 10 verlaufenden Rillen 14 auf. Im Scheitelbereich der Kuppe 12 ist eine sich ausgehend von einem die Zylinderachse "A" umlaufenden Rand 22 in Richtung auf ein der Kuppe 12 abgewandtes axiales Ende 11 des Saugkörpers 10 erstreckende trichterförmige Vertiefung 20 gebildet, welche bei dem in der Zeichnung dargestellten Ausführungsbeispiel der Erfindung rotationssymmetrisch bezüglich der Zylinderachse "A" ausgeführt ist.

Wie besonders deutlich der Fig. 1b) zu entnehmen ist, verjüngt sich der Querschnitt der Vertiefung 20 ausgehend vom Rand 22 in Richtung auf das der Kuppe 12 abgewandte axiale Ende des Saugkörpers 10. Der Durchmesser "d" der Vertiefung 20 an ihrem dem Scheitelbereich abgewandten Rand 22, d. h. der größte Durchmesser der Vertiefung 20 in einer sich senkrecht zur Zylinderachse "A" erstreckenden Richtung, beträgt bei der in der Zeichnung dargestellten Ausführungsform der Erfindung etwa 30 % des Saugkörperdurchmessers.

Wie auch besonders deutlich in Fig. 1b) zu erkennen ist, wird die äußere Begrenzungsfläche des Tampons vollständig von einem Hüllmaterial 50 abgedeckt, welches auch die Begrenzungsfläche der Vertiefung 20 auskleidet. Lediglich im Bereich des Bodens der Vertiefung 20 ist eine Öffnung 60 vorgesehen, welche durch einen Dorn 322 einer Dornanordnung 320 gebildet werden kann, wie im folgenden noch erläutert wird.

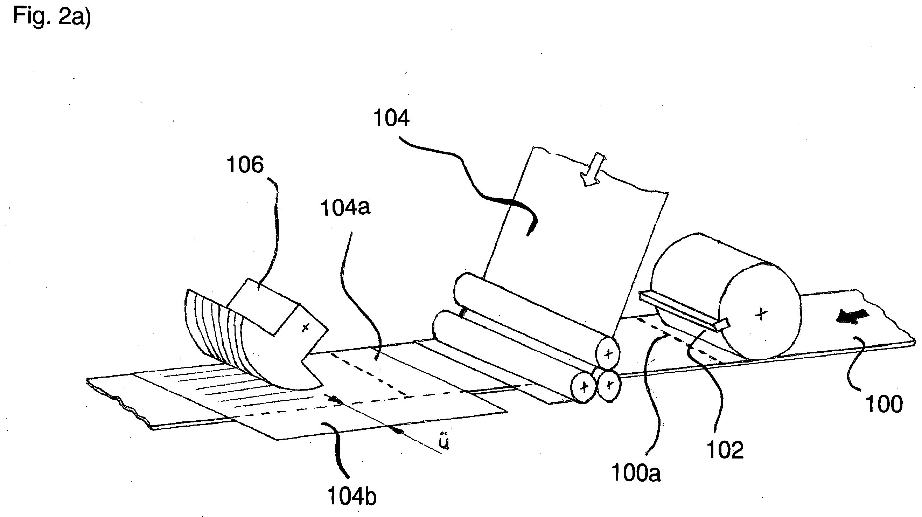

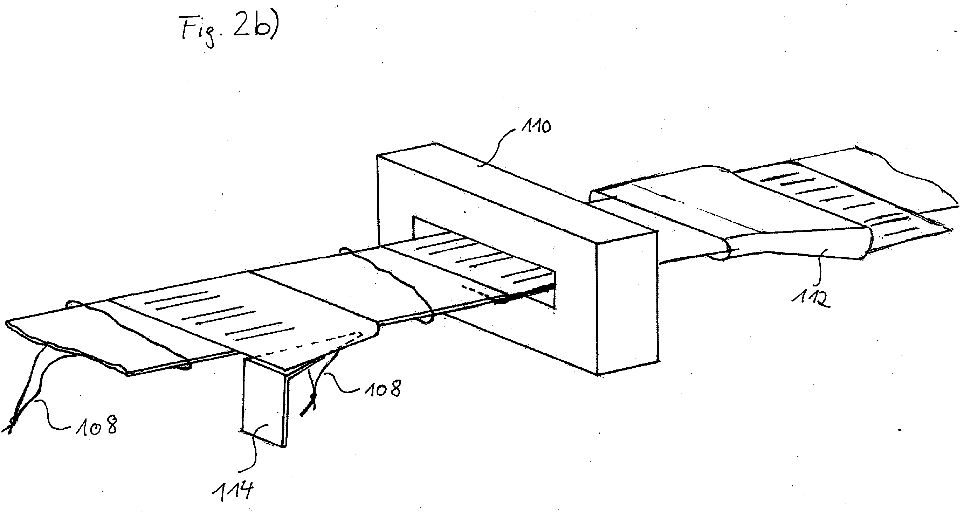

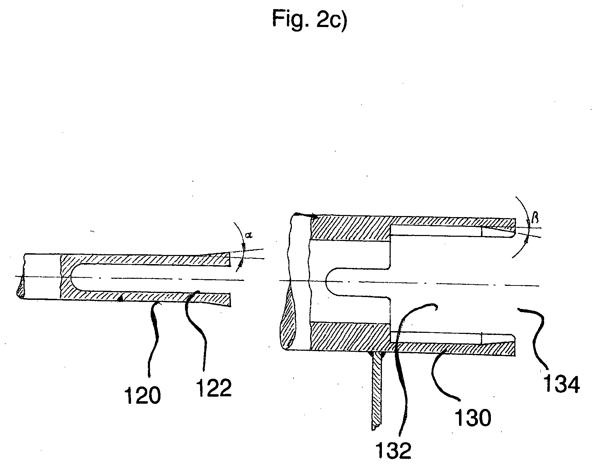

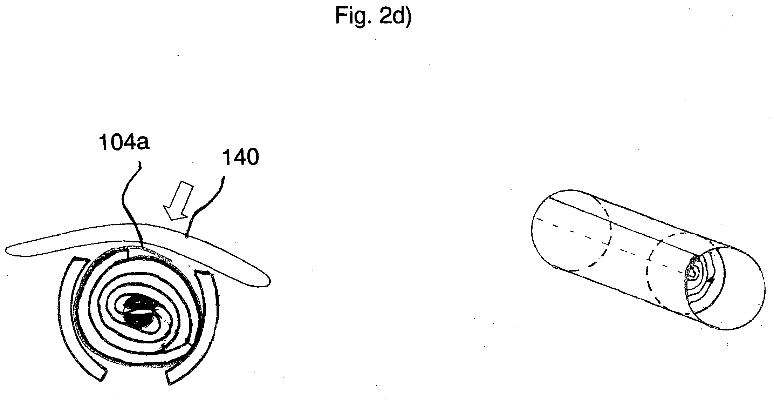

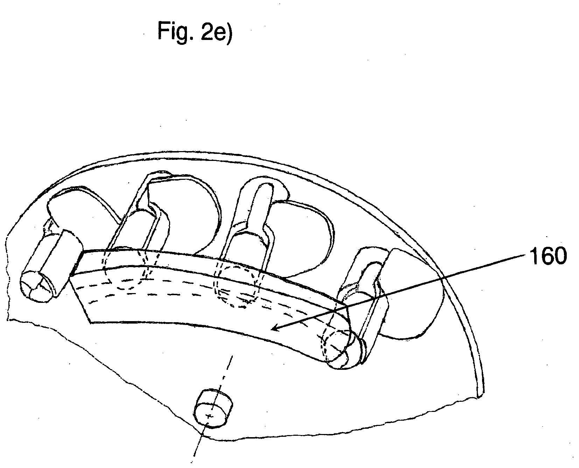

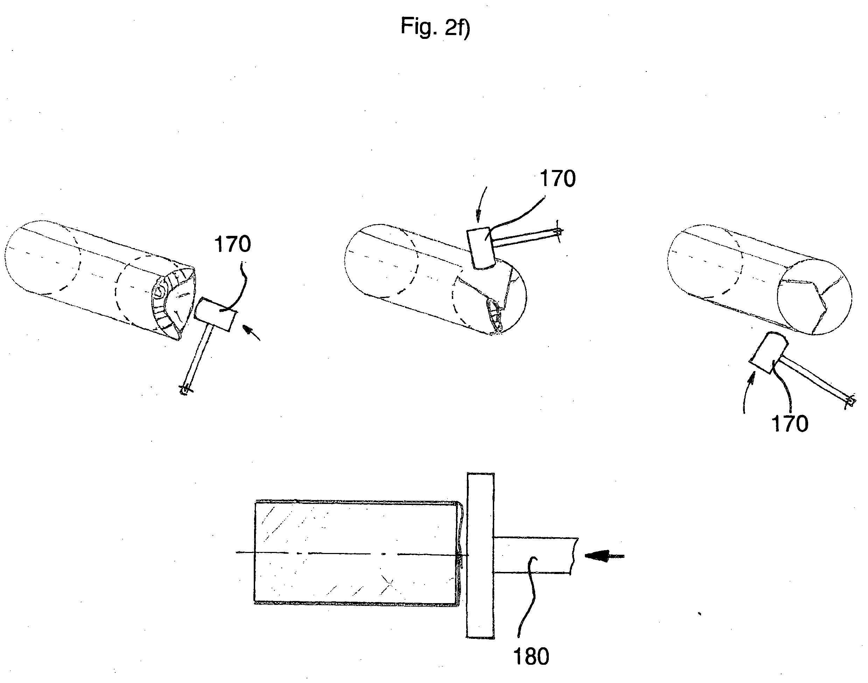

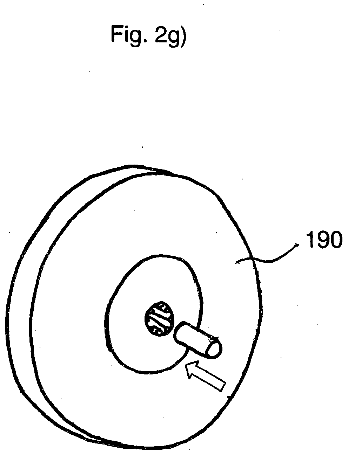

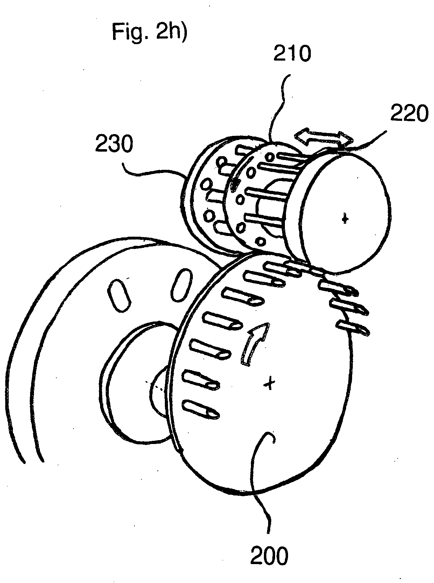

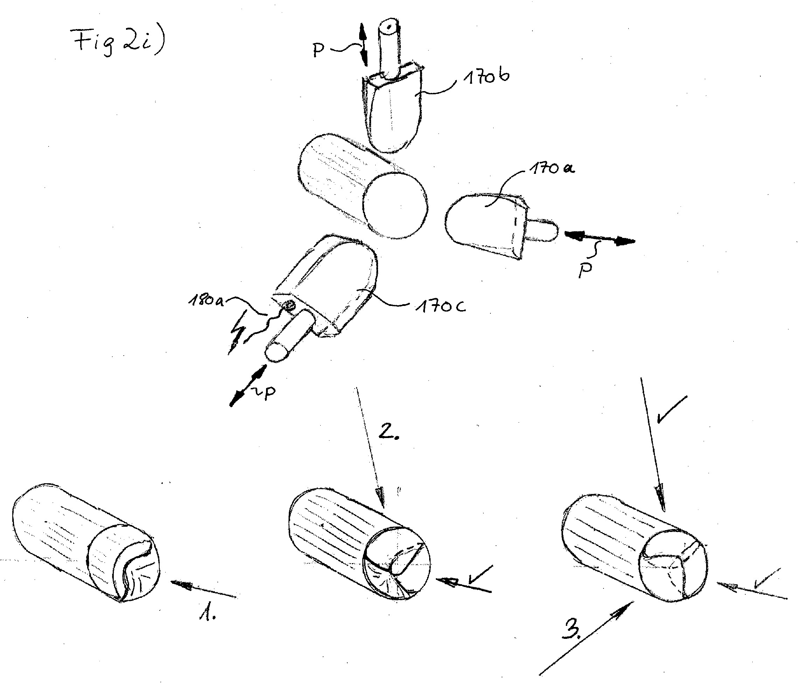

Tampons gemäß Fig. 1 können mit den nachstehend anhand der Fig. 2 erläuterten Vorrichtungen hergestellt werden.

Gemäß Fig. 2 wird bei der Herstellung eines erfindungsgemäßen Tampons zunächst ein Watteband 100 in eine Tamponmaschine eingezogen und mit einem Wattemesser 102 auf die zur Herstellung einzelner Tampons benötigte Länge geschnitten. Die Breite des Wattebands 100 beträgt dabei etwa 40 bis 65 mm. Die einzelnen Wattebandabschnitte, welche zur Herstellung eines Tampons benötigt werden, sind in Fig. 2a) anhand der strichlierten Linien 100a angedeutet. Nach Schneiden des Wattebands 100 zu Wattebandabschnitten mit der benötigten Länge wird ein Hüllvlies 104 mit etwa der halben Länge der Wattebandabschnitte derart versetzt um etwa 50 bis 80 % der Länge eines Wattebandabschnitts unter Druck und Hitze mit Hilfe einer Siegelbacke 106 auf das Watteband 100 aufgesiegelt, daß sich ein Querüberstand 104a über den Wattebandabschnitt ergibt, auf den das Hüllvlies 104 mit Hilfe der Siegelbacke 106 aufgesiegelt ist. Beim Aufwickeln des Wattebands 100 zu einem Vorprodukt des Tampons kommt der Querüberstand 104a auf einem auf dem Watteband 100 aufgesiegelten Bereich des Hüllvlieses 104 zu liegen und kann darauf befestigt werden. Die Breite des Hüllvlieses 104 übersteigt die Breite des Wattebands 100 um 13 bis 63 %. Bei bündiger Anlage des Hüllvlieses 104 an einem Längsrand des Wattebands 100 ergibt sich im Bereich des anderen Längsrands des Wattebands 100 ein Längsüberstand 104b von 13 bis 63 %, insbesondere 20 bis 50 %, der bei der anhand der Zeichnung erläuterten Ausführungsform der Erfindung etwa 8 bis 25 mm beträgt. Der Längsüberstand 104b ist im Bereich des Längsrands des Wattebands 100 gebildet, welcher später die Kuppe 12 des Tampons bildet.

In dem anhand der Fig. 2b) erläuterten Herstellungsschritt wird ein Rückholfaden 108 um das Watteband 100 geschlungen, wobei für jeden Wattebandabschnitt, der später ein Tampon bildet, ein Rückholfaden 108 vorgesehen ist. Wie in Fig. 2b) durch den Pfeil "P" angedeutet, wird das mit dem Rückholfaden 108 ausgestattete Watteband 100 in Richtung auf einen Knoterapparat 110 gefördert, indem die freien Enden des Rückholfadens 108 miteinander verknotet werden. Zur Gewährleistung eines störungsfreien Betriebs des Knoterapparats 110 wird der Längsüberstand 104b vor Erreichen des Knoterapparats 110 mit einem Faltblech 112 umgeschlagen, so daß er bei der anhand der Zeichnung erläuterten Ausführungsform der Erfindung unter dem Watteband 100 zu liegen kommt. Nach Durchlaufen des Knoterapparats 110 wird der Längsüberstand 104b mit einem Führungsblech 114 wieder aufgerichtet, so daß er wieder vom Längsrand des Wattebands 100 herausragt.

Im nächsten Herstellungsschritt wird der mit dem Hüllvlies 104 und dem Rückholfaden 108 ausgestattete Wattebandabschnitt an eine Wickeltrommel übergeben. Die Wickeltrommel umfaßt eine Mehrzahl von Wickeldornen 120 und Wickelhülsen 130. Sowohl die Wickeldornen 120 als auch die Wickelhülsen 130 sind mit Längsschlitzen 122 bzw. 132 ausgestattet, die jeweils etwa kolinear zur Wickelhülsenachse verlaufen (Fig. 2c)). Der mit dem Hüllvlies 104 ausgestattete Wattebandabschnitt wird in den Längsschlitz 122 des in der Wickelhülse 130 drehbar gelagerten Wickeldorns 120 eingeführt. Anschließend wird der Wattebandabschnitt auf den Wickeldorn 120 aufgewickelt. Dabei bleibt der Längsüberstand 104b über den Wattebandabschnitt herausragend bestehen. Das wird ermöglicht, indem die Wickelhülse 130 an ihrem Überstandsende mit einer Öffnung 134 ausgestattet ist, durch welche der Längsüberstand 104b herausragen kann. Der Innendurchmesser der Wickelhülse 130 vermindert sich konisch in Richtung auf dieses Überstandsende 134 der Wickelhülse 130 und/oder der Außendurchmesser des Wickeldorns 120 vergrößert sich in Richtung auf dieses Überstandsende 134. Dadurch wird dem Herausteleskopieren des Wattewickels aus der Wickelhülse 130 entgegengewirkt. Wie in Fig. 2d) schematisch dargestellt, wird der Querüberstand 104a nach Fertigstellung des Wattebandwickels mit Hilfe einer Siegelbacke 140 auf den darunterliegenden und das Watteband 100 abdeckenden Hüllvliesabschnitt aufgesiegelt, um den Wickel so insgesamt zu stabilisieren.

Mit dem Längsüberstand 104b wird die entsprechende Stirnseite des Wattewickels abgedeckt. Dazu kann gemäß der anhand Fig. 2e) veranschaulichten Ausführungsform der Erfindung als kontinuierlich arbeitende Faltanordnung eine Faltkulisse 160 vorgesehen sein, längs der die in den Wickelhülsen 130 aufgenommenen Wattebandwickel an der Wickeltrommel gefördert werden. Durch Drehung der Wickelhülsen 130 mit dem darin aufgenommenen Wickel um die entsprechende Wickelhülsenachse erfolgt mit Hilfe der Faltkulisse 160 automatisch eine kontinuierliche Umfaltung des Längsüberstands 104b über die Stirnfläche des Wattewickels.

Alternativ oder zusätzlich kann gemäß Fig. 2f) eine diskret arbeitende Faltanordnung, z. B. eine Falthämmeranordnung, zum diskreten Umfalten des Längsüberstands 104b auf die Stirnseite des Wattewickels vorgesehen sein. Wie beispielhaft in der Fig. 2f) dargestellt, umfaßt die diskrete Faltanordnung hier drei Falthämmer 170. Es sei bemerkt, daß die Anzahl der Falthämmer je nach Bedarf auch variiert werden kann. Nach Falten des Längsüberstands 104b über die Stirnfläche des Wattebandwickels werden die einander überlappenden Ränder des Längsüberstands 104b mit Hilfe einer Siegeleinheit, hier bspw. mit Hilfe einer Siegelbacke 180, miteinander verbunden, so daß sich eine stabile Anordnung des Wattebandwickels mit dem diesen über den Umfang und eine Stirnseite umhüllenden Hüllmaterial 50 ergibt.

Das so erhaltene Zwischenprodukt mit dem auf die Stirnseite des Wattewickels gefalteten und dort fixierten Hüllmaterial 50 wird anschließend, wie in Fig. 2g) skizziert, an eine geöffnete Wattepresse 190 übergeben. Nach Einschieben des Wattewickels mit dem Hüllmaterial 50 schließt sich die Presse 190 und es wird durch Verpressung ein Rohling erhalten, der ggf. Rillen aufweist, in die das Hüllmaterial 50 eingefaltet ist, und welche eine störungsfreie Ausdehnung des verpreßten Rohlings bei Flüssigkeitsaufnahme ermöglichen. (Die Verpressung des Wickels kann je nach Wahl mittels einer radialen Pressung oder auch einer linearen Pressung vorgenommen werden.) Der Durchmesser des Rohlings wird in Abhängigkeit von der Größe, z. B. mini, normal, super ..., eingestellt.

Nach leichtem Anlüften der Wattepresse 190 wird der damit erhaltene gepreßte und im wesentlichen zylinderförmige Rohling mit einem Ausschieber nach hinten aus der Wattepresse 190 gedrückt und an eine in Fig. 2h) schematisch dargestellte Röhrchentrommel 200 übergeben, derart, daß die mit dem Hüllmaterial 50 verschlossene Stirnseite des Rohlings aus der Röhrchentrommel 200 in einer Kopftrommel 210 mit Stösseln 220 in nachstehend näher erläuterte Formwerkzeuge einer Kuppenformeinrichtung 230 gedrückt werden können, in der schließlich die Vertiefung 20 in der Kuppe 12 gebildet wird. Dazu wird der Rohling mit dem mit dem Hüllmaterial 50 verschlossenen Ende zuerst in die Kopfformer gedrückt. Abhängig von der gewünschten Kuppenform und Ausführung des Tampons sind in der Kuppenformeinrichtung 230 vier bis zwölf gleiche oder auch unterschiedliche Formwerkzeuge eingebaut.

Zusätzlich oder alternativ zu der Falthämmeranordnung gemäß Fig. 2f) kann eine Faltfingeranordnung gemäß Fig. 2i) zum Einsatz kommen. Bei dieser Faltfingeranordnung sind anstelle der Falthämmer 170 drei Faltfinger 170a, 170b und 170c vorgesehen, die, wie durch die Doppelpfeile P angedeutet, hin- und hergehend bewegbar sind. Bei dieser diskreten Faltanordnung wird zunächst ein erster Bereich des Längsüberstands mit dem Faltfinger 170a umgefaltet, dann wird mit dem Faltfinger 170b ein nächster Bereich des Längsüberstands über den bereits umgefaltenen Bereich gefaltet. Schließlich wird der letzte Bereich des Längsüberstands mithilfe des Faltfingers 170c über die mit den Faltfingern 170a und 170b bereits umgefalteten Längsüberstandsbereiche gefaltet. Selbstverständlich werden die einzelnen Faltfinger unmittelbar nach dem damit bewirkten Faltvorgang zurückgezogen, damit der nächste Faltvorgang ungehindert erfolgen kann.

Der Faltfinger 170c ist elektrisch beheizbar, wie bei 180a durch eine Leitung angedeutet. Der Faltfinger 170c, mit dem der letzte Faltvorgang des Längsüberstands bewirkt wird, kann daher gleichzeitig auch als Siegelbacke eingesetzt werden.

Bei der Herstellung erfindungsgemäßer Tampons kann die Breite des Wattebands 40 bis 60 mm betragen. Die Länge des zur Herstellung einzelner Tampons eingesetzten Wattebandabschnitts kann 200 bis 300 mm betragen. Die Länge des auf das Watteband aufgebrachten Hüllmaterialabschnitts beträgt üblicherweise 80 bis 200 mm. Dabei kann ein Querüberstand mit einer Breite von 15 bis 40 mm vorgesehen sein. Das Hüllmaterial wird üblicherweise nicht vollflächig sondern nur über eine Länge von 30 bis 100 mm auf dem Watteband befestigt. Der Wickeldurchmesser des in der Wickelhülse hergestellten Wickels beträgt vor dem Verpressen üblicherweise 20 bis 40 mm. Der zur Herstellung der Abdeckung des Wickels benutzte Längsüberstand des Hüllmaterials kann bei erfindungsgemäßen Tampons eine Breite von 8 bis 25 mm aufweisen. Das bedeutet, daß der Längsüberstand 13 bis 63 % der Watteband- bzw. Saugmaterialbandbreite aufweisen kann.

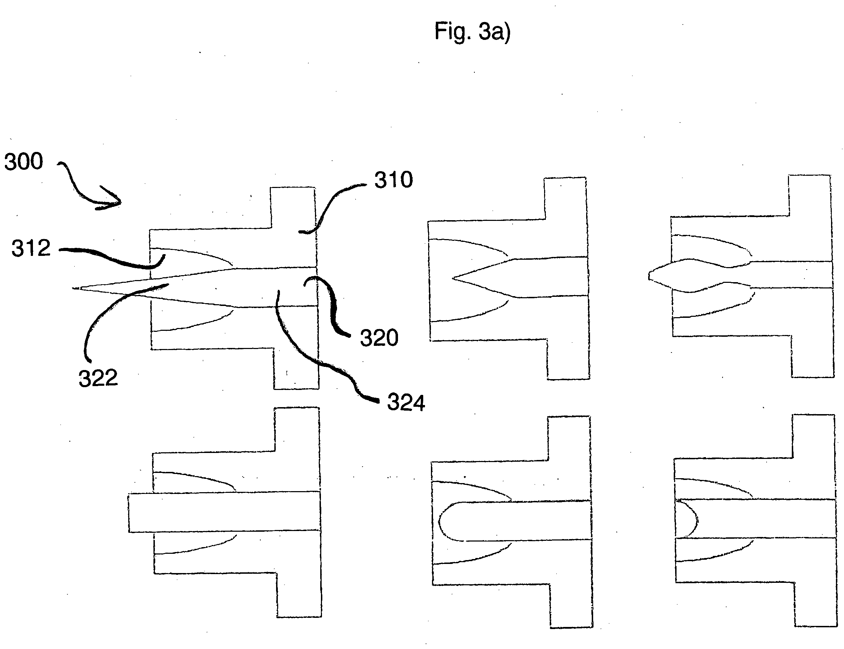

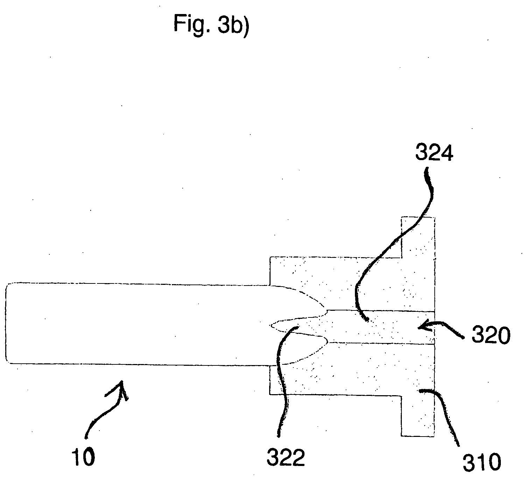

Die in einer erfindungsgemäßen Vorrichtung zur Herstellung erfindungsgemäßer Tampons zum Einsatz kommenden Formwerkzeuge werden nachstehend anhand der Fig. 3 erläutert.

Danach weisen die insgesamt mit 300 bezeichneten Formwerkzeuge einen ringförmigen Mantel 310 und eine Dornanordnung 320 auf. Die Dornanordnung 320 besteht aus einem Dorn 322 und einem Dornträger 324. Der Mantel 310 weist eine innere Begrenzungsfläche 312 mit sich zumindest abschnittweise in axialer Richtung verjüngendem Innendurchmesser auf. Insgesamt ist der Mantel 310 rotationssymmetrisch ausgeführt. Der Dorn 322 ist derart in den Mantel 310 eingeführt, daß zwischen dem den sich verjüngenden Innendurchmesser enthaltenden Bereich der inneren Begrenzungsfläche 312 des Mantels 310 und dem Dorn 322 ein Ringspalt freigelassen ist und der Mantelinnenraum an seinem dem Dorn 322 abgewandten axialen Ende mit dem einstückig mit dem Dorn 322 gebildeten Dornträger 324 verschlossen ist.

Gemäß Fig. 3a) können bei Einsatz identisch ausgeführter Mantelelemente 310 unterschiedlich ausgeführte Dorne 322 zum Einsatz kommen. Dabei können längere, kürzere, dickere oder dünnere Dorne bzw. Dorne mit zylindrischem, bombiertem oder gewelltem Verlauf benutzt werden, wobei die Dornform entsprechend der gewünschten Form der Vertiefung 20 in der Kuppe 12 des Tampons gewählt wird.

Gemäß Fig. 3b) wird der Tamponrohling in axialer Richtung in das Formwerkzeug 300 geführt, so daß der Dorn 322 in axialer Richtung in den Tampon eindringt, während gleichzeitig der äußere Kuppenring gebildet wird. Durch das Eindringen des Dorns 322 wird das die entsprechende Stirnfläche des Tampons abdeckende Hüllmaterial 50 verformt und in die zu bildende Vertiefung 20 hineingedrückt. Dabei kann die Dornform und der Hub des Dorns 322 bei der Ausführung erfindungsgemäßer Verfahren mit Hilfe erfindungsgemäßer Vorrichtungen zum Erhalt erfindungsgemäßer Tampons so gewählt werden, daß das Hüllmaterial 50 von dem Dorn 322 durchstoßen wird, um so das Eindringen von Flüssigkeit in den Tamponkern weiter zu begünstigen, ohne dadurch das Risiko des Ausflusens des Wattewickels nennenswert zu erhöhen.

Bei der in der Zeichnung dargestellten Ausführungsform der Erfindung beträgt das Volumen der Vertiefung 20 ausgehend von dem umlaufenden Rand 22 bis zu dem dem der Kuppe 12 abgewandten axialen Ende 11 des Saugkörpers 10 zugewandten Boden weniger als 4 %, insbesondere etwa 0,54 %, des Gesamtvolumens des Tampons. Dabei kann sich der Durchmesser der Vertiefung 20 ausgehend von einem Randdurchmesser im Bereich von etwa 5 mm über mehrere Stufen verjüngen und die Vertiefung 20 in einer Spitze auslaufen, in der sie ggf. das Hüllvlies 104 durchdringt. Wie besonders deutlich in Fig. 1b) zu erkennen ist, wird ein in einen Kanal mündender Trichter gebildet, durch den die Menstruationsflüssigkeit in den Kern des Saugkörpers 10 eingeleitet werden kann.

Die Erfindung ist nicht auf das anhand der Zeichnung erläuterte Ausführungsbeispiel beschränkt. Vielmehr ist auch daran gedacht, Tampons mit unterschiedlicher Form der Rillen und/oder unterschiedlicher Querschnittsform der Vertiefung herzustellen. Es können Vertiefungen mit beliebigem, insbes. dreieckigem, quadratischem oder rechteckigem Querschnitt hergestellt werden. Das Hüllmaterial kann nicht nur als Hüllvlies, sondern auch als perforierte Hüllfolie od. dgl. ausgeführt werden. Darüber hinaus ist an den Einsatz von gewebten oder gewirkten Hüllmaterialien, z. B. Garne, gedacht.

Bei anderen Ausführungsformen der Erfindung kann die Vertiefung über die Kuppe hinaus in den Saugkörper hineinreichen. In einigen Fällen ist es auch günstig, wenn die Vertiefung nur einen Teil der Kuppe durchsetzt. Zur erfindungsgemäßen Herstellung von Tampons werden Tamponrohlinge in ein erfindungsgemäßes Formwerkzeug eingeführt. Dabei kann die Vertiefung in mehreren Schritten hergestellt werden, indem der Tamponrohling nacheinander in identisch oder unterschiedlich ausgeführte Formwerkzeuge eingeführt wird. Die Formwerkzeuge können in eine dem Tampon gegenüberliegende Stellung gebracht werden, so daß der Tampon durch axiale Relativbewegung zwischen Formwerkzeug und Rohling nacheinander in unterschiedliche Formwerkzeuge eingeführt wird.

Der mit erfindungsgemäßen Vorrichtungen und erfindungsgemäßen Verfahren hergestellte Tampon kombiniert die Vorteile eines zufriedenstellenden Saugvermögens mit einer glatten, im wesentlichen faserfreien Kuppe, welche ein leichteres und angenehmeres Ein- und Ausführen des Tampons ohne das Risiko, daß Watteflusen zurückbleiben, ermöglicht.

## Patentansprüche

1. Verfahren zum Herstellen eines in eine Körperhöhle, insbes. in die weibliche Scheide, einführbaren Tampons mit einem im wesentlichen zylinderförmigen Saugkörper (10), der eine sich in Richtung auf ein axiales Ende (11) des Saugkörpers (10) verjüngende Kuppe (12) aufweist, die mit einer sich in Richtung auf das entgegengesetzte axiale Ende des Saugkörpers (10) erstreckenden Vertiefung (20) in ihrem Scheitelbereich ausgestattet ist, bei dem eine Materialbahn aus saugfähigem Material zu einem Wickel aufgewickelt und der Wickel unter Bildung der Vertiefung (20) zumindest abschnittweise in radialer und/oder axialer Richtung verpreßt wird, **dadurch gekennzeichnet, daß** eine etwa senkrecht zur Wickelachse verlaufende Stirnfläche des Wickels vor der Verpressung vollständig mit einem flüssigkeitsdurchlässigen Hüllmaterial (50) abgedeckt wird und vor dem Wickelvorgang ein Rückholfaden (108) um die Materialbahn geschlungen wird und die von dem Rückholfaden (108) umschlungene Materialbahn einen Knoterapparat (110) durchläuft, wobei der Längsüberstand (104b) vor Durchlaufen des Knoterapparats (110) bezüglich einer etwa kolinear zum Längsrand verlaufenden Faltlinie umgefaltet und nach Durchlaufen des Knoterapparats (110) wieder aufgerichtet wird.

2. Verfahren nach Anspruch 1, **dadurch gekennzeichnet, daß** das Hüllmaterial (50) unter Bildung eines Längsüberstands (104b) über einen etwa senkrecht zur Wickelachse verlaufenden Längsrand der Materialbahn vor dem Wickelvorgang auf die Materialbahn aufgebracht, insbes. aufgesiegelt, wird, wobei sich das Hüllmaterial (50) vorzugsweise über die gesamte Breite der Materialbahn erstreckt und die Breite des Längsüberstands (104b) 13 bis 63 %, vorzugsweise 20 bis 50 %, der Breite der Materialbahn beträgt.

3. Verfahren nach Anspruch 1 oder 2, **dadurch gekennzeichnet, daß** die Materialbahn zu Bahnabschnitten vorgegebener Länge geschnitten und das Hüllmaterial (50) unter Bildung eines Querüberstands (104a) derart über die etwa senkrecht zum Längsrand verlaufende Schnittlinie auf den Bahnabschnitt aufgebracht wird, daß das Hüllmaterial (50) nach dem Wickelvorgang eine Außenschicht des Wickels mit auf dem Hüllmaterial (50) aufliegendem Querüberstand (104a) bildet.

4. Verfahren nach einem der vorhergehenden Ansprüche, **dadurch gekennzeichnet, daß** eine Stirnfläche des Wickels nach dessen Bildung mit dem Längsüberstand (104b) abgedeckt wird.

5. Verfahren nach Anspruch 4, **dadurch gekennzeichnet, daß** der Längsüberstand (104b) nach Bildung des Wickels mit einer Faltanordnung kontinuierlich über die Stirnfläche des Wickels gefaltet wird.

6. Verfahren nach Anspruch 4 oder 5, **dadurch gekennzeichnet, daß** der Längsüberstand (104b) während oder nach Bildung des Wickels mit einer Faltanordnung diskret in ein oder mehreren Schritten über die Stirnfläche des Wickels gefaltet wird.

7. Verfahren nach einem der vorhergehenden Ansprüche, **dadurch gekennzeichnet, daß** übereinanderliegende Bereiche des die Stirnfläche abdeckenden Längsüberstands (104b) miteinander verbunden, insbes. versiegelt, werden.

8. Verfahren nach einem der vorhergehenden Ansprüche, **dadurch gekennzeichnet, daß** nach Verpressung des Wickels ein Formwerkzeug (300), vorzugsweise eine Dornanordnung (324) einer Kuppenformeinrichtung (230), zur Bildung der Vertiefung (20) in die von dem Hüllmaterial (50) abgedeckte Stirnfläche bewegt wird und das Hüllmaterial (50) ggf. durchstößt.

9. Vorrichtung zur Durchführung eines Verfahrens nach einem der Ansprüche 1 bis 8, mit einer Wickeleinrichtung zum Herstellen eines Wickels aus einer Materialbahn aus saugfähigem Material und einer Preßeinrichtung zum Verpressen des Wickels, einer Kuppenformeinrichtung (230) zur Bildung der die Vertiefung (20) aufweisenden Kuppe (12), sowie einer Abdeckeinrichtung zum Abdecken einer Stirnfläche des Wickels mit dem Hüllmaterial (50) vor der Verpressung, eine Zuführeinrichtung zum Aufbringen des Hüllmaterials (50) auf die Materialbahn unter Bildung eines Längsüberstands (104b) und eines Querüberstands (104a), **gekennzeichnet durch** eine zum Umschlingen der Materialbahn mit einem Rückholfaden (108) ausgelegte Umschlingungseinrichtung und den von der Materialbahn zu durchlaufenden Knoterapparat (110) zum Verknoten der Enden des die Materialbahn umschlingenden Rückholfadens (108) sowie einer Falteinrichtung zum Umfalten des Längsüberstands (104b) bezüglich einer etwa parallel zum Längsüberstand (104b) der Materialbahn verlaufenden Faltlinie und eine Rückführeinrichtung zum Aufrichten des Längsüberstands (104b) nach Durchlaufen des Knoterapparats (110).

10. Vorrichtung nach Anspruch 9, **dadurch gekennzeichnet, daß** die Falteinrichtung ein Faltblech (112) aufweist, längs dem der Längsüberstand (104b) des auf der Materialbahn aufliegenden und vorzugsweise damit verbundenen Hüllmaterialbands umgefaltet wird, wenn die Materialbahn an dem Faltblech (112) vorbeigefördert wird und/oder die Rückführeinrichtung ein Führungsblech (114) aufweist, mit dem der umgefaltete Längsüberstand (104b) aufgerichtet wird, wobei der Knoterapparat (110) vorzugsweise zwischen dem Faltblech (112) und dem Führungsblech (114) angeordnet ist.

11. Vorrichtung nach einem der Ansprüche 9 oder 10, **dadurch gekennzeichnet, daß** die Wickeleinrichtung eine Wickelhülse (130) und einen in der Wickelhülse (130) bezüglich der Hülsenachse drehbar gelagerten Wickeldorn (120) aufweist, wobei sowohl die Wickelhülse (130) als auch der Wickeldorn (120) einen sich in Richtung auf die Hülsenachse erstreckenden Aufnahmeschlitz aufweisen, in den die Materialbahn mit dem darauf aufgebrachten Hüllmaterialband einführbar ist, wobei sich der Innendurchmesser der Wickelhülse (130) in Richtung auf ein offenes Überstandsende (134) davon verjüngt und/oder der Außendurchmesser des Wickeldorns (120) sich in Richtung auf das Überstandsende (134) davon vergrößert.

12. Vorrichtung nach einem der Ansprüche 9 bis 11, **dadurch gekennzeichnet, daß** die Abdeckeinrichtung eine kontinuierlich arbeitende Faltanordnung, bspw. eine Faltkulisse (160), aufweist, mit der der aus der Überstandsöffnung herausragende Längsüberstand (104b) derart über die Stirnfläche des Wickels kontinuierlich gefaltet wird, daß die Stirnfläche von dem Hüllmaterial vollständig abgedeckt wird.

13. Vorrichtung nach einem der Ansprüche 9 bis 12, **dadurch gekennzeichnet, daß** die Abdeckeinrichtung eine zum Falten des Längsüberstands (104b) über eine Stirnfläche des Wickels ausgelegte diskret arbeitende Faltanordnung, vorzugsweise eine Falthämmer- und /oder Faltfingeranordnung, insbesondere an ein oder mehreren Stopppositionen der Wickelvorrichtung zur Bildung des Wickels, aufweist.

14. Vorrichtung nach einem der Ansprüche 9 bis 13, **dadurch gekennzeichnet, daß** der Abdeckeinrichtung eine zum Verbinden gegenüberliegender Bereiche des die Stirnfläche des Wickels abdeckenden Längsüberstands (104b) ausgelegte Verbindungseinrichtung, vorzugsweise eine Siegeleinrichtung, zugeordnet bzw. nachgeordnet ist.

15. Vorrichtung nach einem der Ansprüche 9 bis 14, **dadurch gekennzeichnet, daß** die Kuppenformeinrichtung (230) der Preßeinrichtung nachgeordnet ist und ein zur Bildung der Vertiefung (20) in die mit dem Hüllmaterial (50) abgedeckte Stirnfläche des Wickels einführbares Formwerkzeug (300), insbes. eine Dornanordnung (324), aufweist.

16. Vorrichtung nach Anspruch 15, **dadurch gekennzeichnet, daß** die Dornanordnung (324) zum Durchbohren des Hüllmaterials (50) ausgelegt ist.

## Claims

1. A method of producing a tampon that can be introduced into a body cavity, in particular into a woman's vagina, comprising a substantially cylindrical absorbent body (10) which has a summit (12) that tapers towards an axial end (11) of the absorbent body (10), which summit is equipped with a recess (20) extending towards the opposite axial end of the absorbent body (10) in its apex region, a material web made of absorbent material being wound into a winding, and the winding being pressed at least in sections in the radial and/or axial direction such as to form the recess (20), **characterised in that** a front face of the winding running approximately perpendicular to the winding axis is totally covered with a liquid-permeable covering material (50) before the pressing, and before the winding process a retrieval string (108) is looped around the material web and the material web around,which the retrieval string (108) is looped runs through a knotting apparatus (110), the longitudinal projection (104b) being folded around a folding line running approximately co-linearly to the longitudinal edge before passing through the knotting apparatus (110), and being re-aligned after passing through the knotting apparatus (110).

2. The method according to Claim 1, **characterised in that** the covering material (50) is applied, in particular sealed over, the material web before the winding process such as to form a longitudinal projection (104b) over a longitudinal edge of the material web running approximately perpendicular to the winding axis, the covering material (50) preferably extending over the entire width of the material web, and the width of the longitudinal projection (104b) being 13 to 63%, preferably 20 to 50%, of the width of the material web.

3. The method according to Claim 1 or 2, **characterised in that** the material web is cut into web sections of defined length and the covering material (50) is applied to the web section such as to form a transverse projection (104a) over the section line running approximately perpendicular to the longitudinal edge such that the covering material (50) forms an outer layer of the winding with a transverse projection (104a) lying over the covering material (50) after the winding process.

4. The method according to any of the preceding claims, **characterised in that** a front face of the winding is covered after it has been formed with the longitudinal projection (104b).

5. The method according to Claim 4, **characterised in that** the longitudinal projection (104b) is folded with a fold configuration continuously over the front face of the winding after forming the winding.

6. The method according to Claim 4 or 5, **characterised in that** during or after formation of the winding the longitudinal projection (104b) is folded discretely with a fold configuration in one or more steps over the front face of the winding.

7. The method according to any of the preceding claims, **characterised in that** regions of the longitudinal projection (104b) covering the front face and that lie one over the other are connected to one another, in particular sealed.

8. The method according to any of the preceding claims, **characterised in that** after pressing the winding a moulding tool (300), preferably a mandrel arrangement (324) of a summit moulding device (230), is moved to form the recess (20) in the front face covered by the covering material (50) and optionally punctures the covering material (50).

9. An apparatus for implementing a method according to any of Claims 1 to 8, comprising a winding device for producing a winding from a material web made of absorbent material and a pressing device for pressing the winding, a summit moulding device (230) for forming the summit (12) that has the recess (20), and a covering device for covering a front face of the winding with the covering material (50) before pressing, a feed device for applying the covering material (50) to the material web such as to form a longitudinal projection (104b) and a transverse projection (104a), **characterised by** a looping device designed to loop a retrieval string (108) around the material web and the knotting apparatus (110) through which the material web is to pass for knotting the ends of the retrieval string (108) looped around the material web and a folding device for folding the longitudinal projection (104b) around a folding line running approximately parallel to the longitudinal projection (104b) of the material web and a return device for aligning the longitudinal projection (104b) after passing through the knotting apparatus (110).

10. The apparatus according to Claim 9, **characterised in that** the folding device has a folding plate (112) along and around which the longitudinal projection (104b) of the strip of covering material lying over the material web and preferably connected to it is folded when the material web is conveyed past the folding plate (112) and/or the return device has a guide plate (114) with which the folded longitudinal projection (104b) is aligned, the knotting apparatus (110) preferably being disposed between the folding plate (112) and the guide plate (114).

11. The apparatus according to either of Claims 9 or 10, **characterised in that** the winding device has a winding sleeve (130) and a winding mandrel (120) mounted rotatably in the winding sleeve (130) relative to the sleeve axis, both the winding sleeve (130) and the winding mandrel (120) having a receiving slot extending towards the sleeve axis and into which the material web with the strip of covering material applied to it can be introduced, the inside diameter of the winding sleeve (13) tapering away towards an open projection end (134) and/or the outside diameter of the winding mandrel (120) increasing towards the end of the projection (134).

12. The apparatus according to any of Claims 9 to 11, **characterised in that** the covering device has a continuously working fold configuration, for example a folding link (160) with which the longitudinal projection (104b) protruding from the projection opening is continuously folded over the front face of the winding such that the front face is totally covered by the covering material.

13. The apparatus according to any of Claims 9 to 12, **characterised in that** the covering device has a discretely working fold configuration designed to fold the longitudinal projection (104b) over a front face of the winding, preferably a folding hammer and/or folding finger configuration, in particular onto one or more stop positions of the winding apparatus for forming the winding.

14. The apparatus according to any of Claims 9 to 13, **characterised in that** a connection device, preferably a sealing device, designed to connect opposite regions of the longitudinal projection (104b) covering the front face of the winding, is assigned to or follows the covering device.

15. The apparatus according to any of Claims 9 to 14, **characterised in that** the summit moulding device (230) is arranged following the pressing device and has a moulding tool (300), in particular a mandrel arrangement (324), for forming the recess (20) in the front face of the winding covered by the covering material (50).

16. The apparatus according to Claim 15, **characterised in that** the mandrel arrangement (324) is designed to drill through the covering material (50).

## Revendications

1. Procédé de fabrication d'un tampon susceptible d'être introduit dans une cavité corporelle, plus spécifiquement dans le vagin, comprenant un corps absorbant (10) sensiblement cylindrique qui présente une extrémité arrondie (12) qui va en rétrécissant en direction d'une extrémité axiale (11) du corps absorbant (10) et qui est pourvue, à son sommet, d'un creux (20) allongé en direction de l'extrémité axiale opposée du corps absorbant (10), dans lequel une bande de matière absorbante est enroulée pour former un enroulement et l'enroulement est comprimé au moins par sections dans le sens radial et/ou axial, avec formation du creux (20), **caractérisé en ce qu'**une face avant de l'enroulement qui est orientée de manière sensiblement perpendiculaire à l'axe d'enroulement est totalement recouverte, avant la compression, d'un matériau d'enveloppe (50) perméable aux fluides et **en ce que**, avant le processus d'enroulement, un fil de retrait (108) est passé autour, de la bande de matière et la bande de matière entourée du fil de retrait (108) traverse un appareil noueur (110), l'excédent longitudinal (104b) existant en amont du passage dans l'appareil noueur (110) étant plié selon une ligne de pliage orientée de manière sensiblement colinéaire au bord axial et étant redressé en aval du passage dans l'appareil noueur (110).

2. Procédé selon la revendication 1, **caractérisé en ce que** le matériau d'enveloppe (50) est appliqué, ou plus spécifiquement scellé, sur la bande de matière, le long d'un bord longitudinal de la bande de matière qui est orienté de manière sensiblement perpendiculaire à l'axe d'enroulement, avant le processus d'enroulement, avec formation d'un excédent longitudinal (104b), ledit matériau d'enveloppe (50) s'étendant de préférence sur toute la largeur de la bande de matière, la largeur de l'excédent longitudinal (104b) équivalant de 13 à 63 %, de préférence de 20 à 50 % de la largeur de la bande de matière.

3. Procédé selon la revendication 1 ou 2, **caractérisé en ce que** la bande de matière est découpée en sections de bande de longueur prédéfinie et le matériau d'enveloppe (50) est appliqué sur la section de bande en formant un excédent transversal (104a), le long de la ligne de découpe orientée de manière sensiblement perpendiculaire au bord longitudinal de telle manière que, suite au processus d'enroulement, le matériau d'enveloppe (50) forme une couche extérieure de l'enroulement qui présente un excédent transversal (104a) reposant sur le matériau d'enveloppe (50).

4. Procédé selon l'une quelconque des revendications précédentes, **caractérisé en ce qu'**une face avant de l'enroulement est recouverte, après sa formation, de l'excédent longitudinal (104b).

5. Procédé selon la revendication 4, **caractérisé en ce que**, après formation de l'enroulement, l'excédent longitudinal (104b) est plié de manière continue sur la face avant de l'enroulement à l'aide d'un dispositif de pliage.

6. Procédé selon la revendication 4 ou 5, **caractérisé en ce que**, pendant ou après la formation de l'enroulement, l'excédent longitudinal (104b) est plié de manière discontinue en une ou plusieurs étapes sur la face avant de l'enroulement à l'aide d'un dispositif de pliage.

7. Procédé selon l'une quelconque des revendications précédentes, **caractérisé en ce que** des zones superposées de l'excédent longitudinal (104b) recouvrant la face avant sont liées, plus spécifiquement scellées, les unes aux autres.

8. Procédé selon l'une quelconque des revendications précédentes, **caractérisé en ce que**, après la compression de l'enroulement, un outil de façonnage (300), de préférence un dispositif à mandrin (324) d'un dispositif de façonnage d'extrémité arrondie (230), est introduit dans la face avant recouverte du matériau d'enveloppe (50) afin de former le creux (20), et perfore éventuellement le matériau d'enveloppe (50).

9. Dispositif permettant de mettre en oeuvre un procédé selon l'une des revendications précédentes 1 à 8, comprenant un dispositif enrouleur pour la fabrication d'un enroulement à partir d'une bande de matière absorbante, un dispositif de compression permettant de comprimer l'enroulement, un dispositif de façonnage d'extrémité arrondie (230) permettant de former l'extrémité arrondie (12) présentant ledit creux (20), ainsi qu'une unité de recouvrement permettant de recouvrir une face avant de l'enroulement avec le matériau d'enveloppe (50) avant la compression, un dispositif d'amenée permettant, d'apporter le matériau d'enveloppe (50) sur la bande de matière en formant un excédent longitudinal (104b) et un excédent transversal (104a), **caractérisé par** un dispositif de mise en place de fil permettant d'entourer la bande de matière avec un fil de retrait (108) et par l'appareil noueur (110) destiné à être traversé par la bande de matière et permettant de nouer l'extrémité du fil de retrait (108) entourant la bande de matière, ainsi que par un dispositif de pliage permettant de plier l'excédent longitudinal (104b) selon une ligne de pliage orientée de manière sensiblement parallèle à l'excédent longitudinal (104b) de la bande de matière et par un dispositif de renvoi permettant de redresser l'excédent longitudinal (104b) après le passage dans l'appareil noueur (110).

10. Dispositif selon la revendication 9, **caractérisé en ce que** le dispositif de pliage présente une plaque de pliage (112) le long de laquelle l'excédent longitudinal (104b) de la bande de matériau d'enveloppe appliquée sur la bande de matière, et de préférence liée à celle-ci, est replié lorsque la bande de matière est transportée le long de la plaque de pliage (112) et/ou le dispositif de renvoi présente une plaque de guidage (114) à l'aide de laquelle l'excédent longitudinal (104b) replié est redressé, ledit appareil noueur (110) étant situé de préférence entre la plaque de pliage (112) et la plaque de guidage (114).

11. Dispositif selon l'une des revendications précédentes 9 et 10, **caractérisé en ce que** le dispositif enrouleur présente une douille d'enroulement (130) et un mandrin d'enroulement (120) disposé rotatif dans la douille d'enroulement (130) par rapport à l'axe de la douille, ladite douille d'enroulement (130) ainsi que le mandrin d'enroulement (120) présentant une fente de réception qui s'étend dans le sens de l'axe de la douille et dans laquelle peut être introduite la bande de matière pourvue de la bande de matériau d'enveloppe appliquée sur celle-ci, le diamètre intérieur de la douille d'enroulement (130) allant en rétrécissant dans le sens d'une extrémité pour excédent ouverte (134) de celle-ci et/ou le diamètre extérieur du mandrin d'enroulement (120) allant en grossissant dans le sens de l'extrémité pour excédent (134) de celle-ci.

12. Dispositif selon l'une des revendications précédentes 9 à 11, **caractérisé en ce que** l'unité de recouvrement présente un dispositif de pliage à fonctionnement continu, par exemple une coulisse de pliage (160), avec lequel l'excédent longitudinal (104b) faisant saillie hors de l'ouverture pour excédent est plié en continu sur la face avant de l'enroulement, de telle manière que la face avant se retrouve totalement recouverte du matériau d'enveloppe.

13. Dispositif selon l'une des revendications précédentes 9 à 12, **caractérisé en ce que** l'unité de recouvrement présente un dispositif de pliage à fonctionnement discontinu permettant de plier l'excédent longitudinal (104b) sur une face avant de l'enroulement, de préférence un dispositif à marteaux de pliage et/ou à doigts de pliage, notamment en une ou plusieurs positions d'arrêt de l'unité d'enroulement destinée à former l'enroulement.

14. Dispositif selon l'une des revendications précédentes 9 à 13, **caractérisé en ce qu'**une unité de liaison, de préférence une unité de scellage, conçue pour lier des zones opposées de l'excédent longitudinal (104b) recouvrant la face avant de l'enroulement, est associée à l'unité de recouvrement ou située en aval de celle-ci.

15. Dispositif selon l'une des revendications précédentes 9 à 14, **caractérisé en ce que** le dispositif de façonnage d'extrémité arrondie (230) est situé en aval du dispositif de compression et présente un outil de façonnage (300), plus spécifiquement un dispositif à mandrin (324), susceptible d'être introduit dans la face avant de l'enroulement recouverte du matériau d'enveloppe (50) afin de former le creux (20),

16. Dispositif selon la revendication 15, **caractérisé en ce que** le dispositif à mandrin (324) est conçu pour percer le matériau d'enveloppe (50).
